# EUROPEAN PATENT APPLICATION

(11) **EP 2 058 662 A1**
(43) Date of publication of application: **13.05.2009**
(21) Application number: 07118656.3
(22) Date of filing: 17.10.2007
(51) Int. Cl.: G01N 33/546, G01N 33/543, G01N 33/68, G01N 33/569, G01N 33/574

(54) **Diagnosis of diseases by measuring cell mediated extracellular matrix degradation**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The present invention relates to the field of diagnosing diseases using cell-based assays. Particularly, the present invention relates to using the effect of cells on extracellular matrix stability for diagnosing coronary artery diseases.

## Description

### SUBJECT OF THE INVENTION

The present invention relates to the field of diagnosing diseases using cell-based assays. In particular, the present invention pertains to the field of diagnosing coronary artery diseases.

### BACKGROUND OF THE INVENTION

It is well known that growth factors are involved in the development of a variety of diseases including coronary artery disease, diabetes, and various types of cancer such as e.g. breast cancer.

As a consequence, attempts have been made to elucidate the precise function of growth factors but also other disease-related factors during disease development.

Concurrently, with the increasing knowledge of the role of e.g. growth factors during development of coronary artery diseases or different types of cancers, attempts have been to use these factors as a diagnostic marker, either for ongoing disease development or the future-likely occurrence of the disease in question. One approach typically undertaken in this respect is to determine whether expression of a disease-specific factor such as a growth factor is up-regulated or down-regulated compared to a control individual that is known not to suffer from the disease in question.

While these approaches allowed for significant improvements in the diagnosis and therapy of a multitude of diseases, there is a continuing need for diagnostic methods/test systems that allow a physician to decide on the disease state of a patient.

### OBJECT AND SUMMARY OF THE INVENTION

It is one objective of the present invention to provide novel approaches for diagnosing different types of diseases.

It is a further objective of the present invention to provide methods that allow obtaining data that can then be used in diagnosing a variety of diseases.

These objectives as well as others, which will become apparent from the ensuing description, are attained by the subject matter of the independent claims. Some of the preferred embodiments of the present invention are defined by the dependent claims.

In one embodiment the present invention relates to a method of diagnosing and/or staging a disease comprising at least the following steps:
a) Providing at least one carrier structure wherein said carrier structure comprises an extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold;
e) Deciding on the occurrence and/or stage of the disease in question based on the data obtained in step d).

Deciding on the occurrence and/or the grade of the disease in step e) may be undertaken by different approaches. Thus, one may repeat steps a) to d) for a patient at different times to establish a pattern for the extent of interaction. If one observes a change in the extent of interaction over time this may be an indication of ongoing disease development.

In addition or alternatively one may compare the data obtained in step d) with a suitable control of healthy subjects. In this embodiment the invention relates to a method of diagnosing and/or staging a disease comprising at least the following steps:
a) Providing at least one carrier structure wherein said carrier structure comprises an extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold;
e) Providing at least one control sample of a human or animal being not suffering from the disease in question wherein said control sample comprises at least one cell that is capable of specifically interacting with said at least one probe molecule;
f) Contacting said at least one control sample with said at least one carrier structure;
g) Determining the extent of the influence of said at least one cell within said at least one control sample being associated with said at least one probe molecule on the stability of the ECM scaffold;
h) Comparing the extent of influence as determined in steps d) and g);
i) Deciding on the occurrence of the disease in question based on the comparison made in step h).

In one embodiment all of the aforementioned steps are performed outside the human or animal body.

Another embodiment of the present invention relates to a method of data acquisition comprising at least the following steps:
a) Providing at least one carrier structure wherein said carrier structure comprises a extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold.

One may use the method of data acquisition again to either monitor a subject over a period of time by repeating steps a) to d) and/or to compare the data with those obtained for a control. In this latter case, the invention relates to a method of data acquisition comprising at least the following steps:
a) Providing at least one carrier structure wherein said carrier structure comprises a extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold;
e) Providing at least one control sample of a human or animal being not suffering from the disease in question wherein said control sample comprises at least one cell that is capable of specifically interacting with said at least one probe molecule;
f) Contacting said at least one control sample with said at least one carrier structure;
g) Determining the extent of the influence of said at least one cell within said at least one control sample being associated with said at least one probe molecule on the stability of the ECM scaffold;
h) Comparing the extent of influence as determined in steps d) and g).

In one embodiment all of the aforementioned steps are performed outside the human or animal body.

Typically said carrier structure may be selected to be a microcarrier structure such as a microcarrier bead having a diameter of about 20 µm to about 1000 µm. A size of about 75 µm to about 350 µm, of about 100 µm to about 200 µm and about 150 µm can be preferred.

The methods in accordance with the invention can be used to diagnose a variety of diseases with coronary artery diseases, diabetes, different types of cancers, and inflammatory diseases such as rheumatoid arthritis constituting typical examples. Some of the preferred embodiments relate to the diagnosis of coronary artery diseases, including diseases such as atherosclerosis.

In a typical embodiment of the present invention, said at least one probe molecule will be selected from the group comprising small molecules, peptides, glycopeptides, proteins, glycoproteins, receptors, receptor ligands, antibodies, growth factors, hormones, cytokines, chemokines, cell adhesion molecules, vitamins, nucleic acids and derivatives thereof, lipids and glycans. A particularly preferred embodiment may relate to probe molecules, which are growth factors such as e.g. vascular endothelial growth factor (VEGF), vascular cell adhesion molecule 1 (VCAM-1).

The cell (type) the association of which with the probe molecule is to be determined will depend to some extent on the disease to be analysed as well as the probe molecules being used. The cells in accordance with the invention will typically be cells that are known to secrete extracellular matrix proteases. Typical examples of cells that are investigated in embodiments of the present invention include embryonic, fetal or adult stem cells, progenitor cells, blood cells, fibroblasts, tumour cells and urinal cells. In a preferred embodiment, the cells are derived from blood cells and include monocytes, macrophages, and neutrophils.

A particularly preferred embodiment relates to methods of diagnosing and methods of data acquisition as outlined above in which the at least one probe molecule is VEGF or VCAM-1, in which the at least one cell (type) which is analysed are monocytes and in which the disease to be diagnosed is arteriolosclerosis or atherosclerosis.

The extracellular matrix (ECM) scaffold may preferably be made from proteins including collagen, fibronectin, fibrins, laminins, elastins, nidogens, basal and basement membrane fractions, matrigel, hemicellulose, pectin and extension.

In one aspect of the invention both the ECM scaffold as well as the probe molecule may be covalently attached to the carrier structure. In another aspect, both the ECM scaffold and the probe molecules may be associated with the carrier structure but not covalently attached thereto. Other embodiments of the invention are that the ECM scaffold is associated with but not covalently bound to the carrier structure while the probe molecule is covalently attached thereto and vice versa.

The influence of the cell on the stability of the ECM scaffold can be measured using different approaches. In one embodiment of the present invention the ECM scaffold may be labeled with the detectable marker so that a degradation of the ECM scaffold by the cells will become apparent by a change in the detectable marker. Typical fluorescent markers can include Cy5, Cy3, Texas Red, FITC, Attodye, Cydye, Alexa647, Alexa 488, Alexa 546, Alexa 546, Alexa 594, Alexa 633, GFP, YFP, CFP and dsRED.

In one approach a Förster Resonance Energy Transfer (FRET) approach may be undertaken to study the stability of the ECM matrix scaffold by labeling the ECM matrix scaffold with donor and acceptor fluorophores. In another approach, the probe molecule and the ECM scaffold may be labeled with donor and acceptor fluorophores respectively or vice versa.

In one of the preferred embodiments of the present invention, determination of the influence on the ECM scaffold will involve repeating this analysis for different amounts of ECM scaffold being associated with the carrier structure. This will allow to determine for a given sample the extent by which cells of the sample bind to the carrier structures and degrade the ECM scaffold depending on the amount of ECM being associated with the carrier structure resulting in a profile of extent of degradation/carrier structure versus amount of ECM/carrier structure. Additionally or alternatively the analysis may be undertaken for a constant amount of ECM scaffold and a constant number of cells but for different time points.

In some embodiments of the present invention, these profiles may be compared for a human or animal being suspected of suffering from the disease in question and a control human or animal subject which is known not to suffer from the disease so that a decision as to the ongoing development of the disease or likely future occurrence of the disease is possible on the basis of the profiles described above.

Other embodiments of the present invention will become apparent from the detailed description hereinafter.

### FIGURE LEGENDS

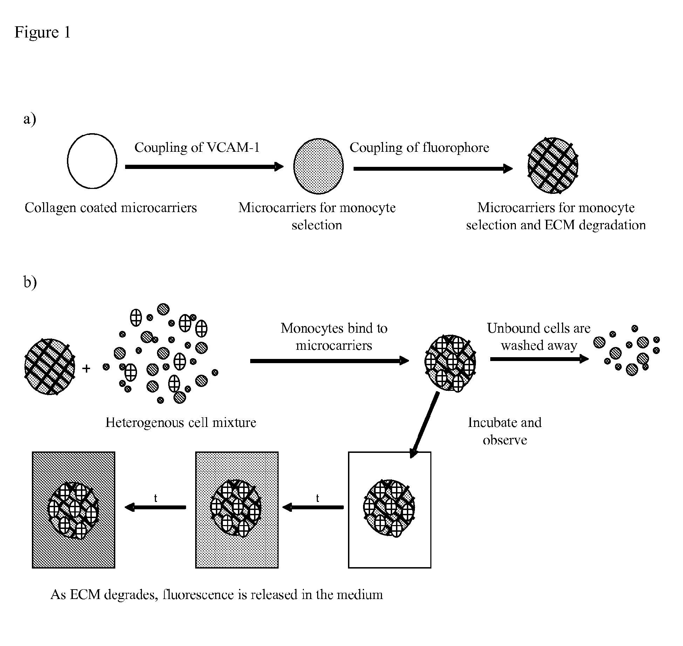

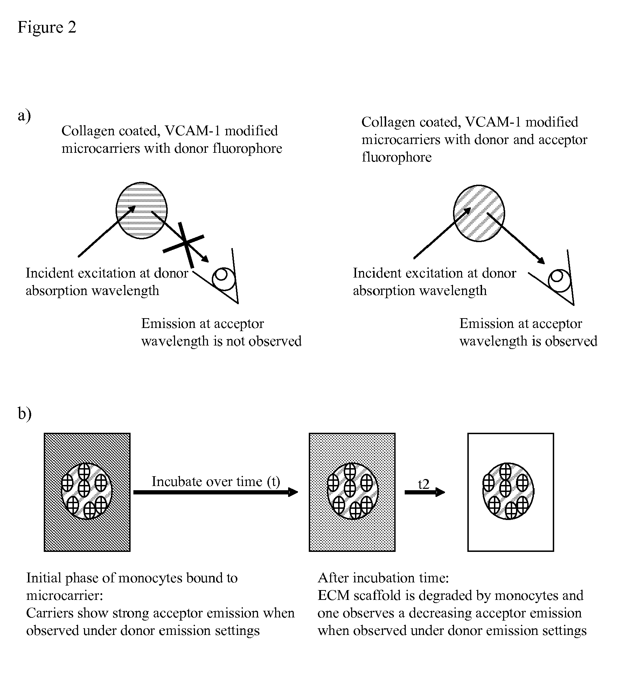

Fig. 1 depicts schematically the approach of selecting cells and detecting their ECM protease activity. a) Collagen coated microcarrier beads are functionalized with vascular cell adhesion molecule-1 (VCAM-1) and subsequent conjugation of a fluorophore to the collagen coated microcarriers. b) depicts incubation of VCAM-1 functionalized and collagen-coated microcarriers with a sample and subsequent binding of monocytes to the beads. Further it is illustrated how fluorescence changes over time as a function of the activity of the protease activity of the monocytes that degrade the ECM scaffold. Fig. 2 depicts the use of FRET for detecting ECM scaffold degradation. a) depicts the difference between collagen coated microcarrier beads when being labeled either only with a donor fluorophore or both with a donor and an acceptor fluorophore. b) depicts how incubation of such microcarrier beads with monocytes and of the adherence of the cells lead to a change in the emission intensity at the acceptor wavelength as a function of the degradation of the ECM scaffold.

### DETAILED DESCRIPTION OF THE INVENTION

For some diseases it is known that disease progression involves the recruiting of specific types of cell. For example, the formation of atherosclerosis is initially characterized by the entry of monocytes into the arterial endothelium. These monocytes later differentiate into macrophages and by phagocytosis begin to accumulate modified and oxidized lipoproteins leading to the eventual formation of foam cells, a hallmark component of atherosclerotic plaques. Under specific conditions the plaque can rupture (thrombosis) and subsequent clotting can lead to a blockage in blood flow, i.e. an infarction (a heart attack or stroke).

The inventors of the present invention have surprisingly found that one can use the matrix metalloproteinase activity of cells such as monocytes and macrophages in the context of diagnosing diseases that are characterized by recruiting these cell types to body sites where disease development progresses.

Before some of the embodiments of the present inventions are described in more detail, the following definitions are introduced.

As used in this specification and in the appended claims, the singular forms of "a" and "an" also include the respective plurals unless the context clearly dictates otherwise. Thus, the term "a probe molecule" can include more than one probe molecule, namely two, three, four, five etc. probe molecule.

The terms "about" or "approximately" in the context of the present invention denotes an interval of accuracy that the person skilled in the art will understand to still ensure the technical effect of the feature in question. The term typically indicates deviation from the indicated numerical value of +/- 10%, and preferably +/- 5%.

It is to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to disclose a group, which preferably consists only of these embodiments.

Further definitions of terms will be given in the context of which the terms are used.

In one embodiment the present invention relates to a method of diagnosing and/or staging a disease comprising at least the following steps:
a) Providing at least one carrier structure wherein said carrier structure comprises an extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold;
e) Deciding on the occurrence and/or stage of the disease in question based on the data obtained in step d).

Deciding on the occurrence and/or the grade of the disease in step e) may be undertaken by different approaches. Thus, one may repeat steps a) to d) for a patient at different times to establish a pattern for the extent of interaction. If one observes a change in the extent of interaction over time this may be an indication of ongoing disease development.

In addition or alternatively one may compare the data obtained in step d) with a suitable control of healthy subjects. In this embodiment the invention relates to a method of diagnosing and/or staging a disease comprising at least the following steps:
a) Providing at least one carrier structure wherein said carrier structure comprises an extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold;
e) Providing at least one control sample of a human or animal being not suffering from the disease in question wherein said control sample comprises at least one cell that is capable of specifically interacting with said at least one probe molecule;
f) Contacting said at least one control sample with said at least one carrier structure;
g) Determining the extent of the influence of said at least one cell within said at least one control sample being associated with said at least one probe molecule on the stability of the ECM scaffold;
h) Comparing the extent of influence as determined in steps d) and g);
i) Deciding on the occurrence of the disease in question based on the comparison made in step h).

In one embodiment all of the aforementioned steps are performed outside the human or animal body.

In another embodiment, the present invention relates to a method of data acquisition comprising at least the following steps:
a) Providing at least one carrier structure wherein said carrier structure comprises a extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold.

One may use the method of data acquisition again to either monitor a subject over a period of time by repeating steps a) to d) and/or to compare the data with those obtained for a control. In this latter case, the invention relates to a method of data acquisition comprising at least the following steps:
a) Providing at least one carrier structure wherein said carrier structure comprises a extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold;
e) Providing at least one control sample of a human or animal being not suffering from the disease in question wherein said control sample comprises at least one cell that is capable of specifically interacting with said at least one probe molecule;
f) Contacting said at least one control sample with said at least one carrier structure;
g) Determining the extent of the influence of said at least one cell within said at least one control sample being associated with said at least one probe molecule on the stability of the ECM scaffold;
h) Comparing the extent of influence as determined in steps d) and g).

In one embodiment all of the aforementioned steps are performed outside the human or animal body.

The specifics of the aforementioned method of diagnosis and method of data acquisition will be discussed hereinafter together.

The carrier structures to be used in the present invention will typically take a 3-dimensional form as this allows the carrier structures to make an intimate contact with the cells that are present within the sample and that should be detected using the methods in accordance with the invention.

The microcarrier structures may have any form that is suitable for the purposes of the present invention. They may thus have a rectangular shape, a triangular shape etc. Beads with round or substantially round form can be preferred according to the present invention. Thus, the carrier structures of the present invention in a preferred embodiment will typically have a round, elliptical or spheroid form.

While the size of the carrier structures is not critical, it can be preferred to use carrier structures that are in the micron size range. These carrier structures will be designated as microcarrier structures. Typically, microcarrier structures will have a diameter in the range of approximately 20 µm to about 1000 µm Diameters in the range of approximately 75 µm to approximately 300 µm may be preferred, as may be diameters in the range of approximately 100 µm to about 200 µm. However, diameters in the range of 120 µm to about 1800 µm or about 150 µm may also be preferred. The size may be selected such that numerous cells (10-100) can uniformly coat one carrier structure. If such microcarrier structures take the aforementioned preferred round, elliptical or spheroid form they may be designated as microcarrier beads.

There are no specific requirements to the components or the interior structure of the microcarrier structures or beads except that the structures or beads must be capable of allowing a probe molecule to associate therewith and must be capable of allowing cells to associate with the probe molecules.

With respect to the aforementioned requirements, e.g. microcarrier beads may be produced from materials such as polystyrene, polydivnylbenzene and polymethylmethacrylate and biodegradable polymers such as poly-lactides, polyclycolides, polycaprolacton and copolymers thereof.

Such beads are in principle commercially available from e.g. SoloHill Engineering, Inc (Ann Arbor, Michigan, USA).

The beads may be further modified, e.g. coated in order to ensure adherence of probe molecules and/or to allow for association of a certain type of cell (see below).

The person skilled in the art is familiar with the production of microcarrier beads. Thus, one may obtain these beads from aforementioned commercial sources or produce them according to the protocols being described in e.g. Gu et al (J. Control Release (2004), 96.3:463-472), Li et al (Acta Pharmacol. Sin. (2006) 27.6:754-759), Norrgren et al (Dev. Biol. Stand. (1983), 55:43-51) or Tatard et al (Biomaterials (2005), 26.17:3727-3737). Thus, the bead technology to create both e.g. microcarrier beads as well as probe covered-beads is known to the skilled person.

Microcarrier beads can e.g. be synthesized e.g. by controlled emulsification techniques such as submerged ink-jet printing.

If a microcarrier bead is to be produced from a polymer such as polystyrene, a polymer solution in a solvent such as dichloromethane can be ink-jetted into an aqueous phase containing a stabilizer for the monodisperse emulsion that is formed. To this end, one can use e.g. polyvinyl alcohol but also proteins could be used to achieve efficient stabilization. Subsequently polymer beads with a narrow size distribution are formed upon removal of the solvent.

If one intends to incorporate e.g. peptides/proteins or nucleic acids as probes, which can be water soluble, a double emulsion technique can be used. To this end, the water-soluble peptides and/or proteins may be first dissolved in an aqueous phase and this aqueous phase is emulsified in the polymer solution. If necessary or desired, additional stabilizers such as e.g. Pluronic can be added. This first emulsion is then ink-jetted into a second aqueous phase and beads are again formed upon removal of the solvent.

The term "extracellular matrix scaffold" in the context of the present invention relates to an assembly of at least one protein as it is typically found in the extracellular matrix of cells. Thus, the extracellular matrix scaffold in accordance with the present invention may be made from proteins including collagen, fibronectin, fibrins, laminins, elastins and nidogens or combinations thereof. In one embodiment the extracellular matrix scaffold may be made e.g. from a layer of collagen. Thus, a carrier structure in accordance with the present invention comprising an extracellular matrix scaffold may in a preferred embodiment be e.g. a microcarrier bead with a collagen layer being disposed thereon. Such carrier structures are commercially available from e.g. Solohill (www.solohill.com). The person skilled in the art is aware how to produce and dispose such extracellular matrix scaffolds on carrier structures. The thickness of such an ECM layer can be on the order of nanometers to micrometers.

A person skilled in the art is also familiar how to functionalize e.g. the surface of a microcarrier bead with the ECM scaffold proteins.

Modification of a microcarrier bead with an ECM scaffold protein may be achieved by covalent or non-covalent interaction between the microcarrier bead and the ECM scaffold protein. To this end, one may e.g. coat the microcarrier bead with a self assembling polymer. Such a self-assembling polymer may e.g. be polyethylenglycol. The polymers may be further functionalized to introduce chemical functionalities that allow e.g. covalent coupling of collagen to the surface of the microcarrier bead.

If ECM scaffold forming proteins such as collagen are used and should be covalently coupled to the microcarrier beads, beads may be used that provide functional groups such as carboxyl groups, amine groups, hydroxyl groups, sulfhydryl groups etc. These groups may then be cross-linked either directly to the ECM scaffold forming proteins or one may use a linker that may be homo-or hetero-bifunctional.

Thus, microcarrier beads may be activated for providing a chemical linkage to the ECM scaffold forming proteins by e.g. coating the beads with a polymer having e.g. acyl fluoride functionalities. Other covalent attachment chemistries are also applicable but not limited to anhydrides, epoxides, aldehydes, hydrazides, acyl azides, aryl azides, diazo compounds, benzophenones, carbodiimides, imidoesters, isothiocyanates, NHS esters, CNBr, maleimides, tosylates, tresyl chloride, maleic acid anhydrides and carbonyldiimidazole.

For functionalising of microcarrier beads with e.g. collagen, one can rely on typical polystyrene beads of approximately 150 µm diameter. Collagen can then be coupled to the surfaces of such beads, which have been functionalised with a reactive group such as e.g. succinimide esters.

Attachment of the ECM scaffold on the carrier structures may be also undertaken by non-covalently attaching the ECM scaffold forming proteins to the carrier structures.

As has been mentioned above, the methods in accordance with the present invention rely on carrier structures and preferably microcarrier structures or beads, which are associated with at least one probe molecule.

Such a probe molecule may be any type of molecule that is capable of specifically interacting with a certain cell type. Typically, probe molecules by their native function may act as a ligand for a cell-surface receptor of the cells to detected.

Thus, probe molecules may be selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycans. However, the probe molecules may be identical to ECM scaffold proteins.

Growth factors and cell adhesion molecules may be preferred as probe molecules. Typical growth factors include e.g. human growth factor, human nerve growth factor, platelet derived growth factor, TGFβ, EGF, VEGF, TNF-a,TNF-b, FGFs, TGF-a, Epo, IGF-1, IGF-11, IL-1, IL-2, IL-6, IL-8, INF-g and CSFs as well as integrins and CAMs including VLA-1 to VLA-6, LPAM-1, LFA-1, CR3, CR4, leukointegrin, collagen, laminin, fibronectin, VCAM-1, MAdCAM-1, E-cadherin, ICAM-1, ICAM-2, ICAM-3, C3b, C4b. Other growth factors include CCL 1-28, CXCL 1-16, XCL1, XC:2, CX3CL1, IL 1-22, INF α, β and y, M-CSF, G-CSF, GM-CSF, MIF, any recognized CD marker of hematopoetic cells (CD 1-339), PDGF, NGF, TPO, GDF-8, GDF-9, bFGF, HGF, FGF, DII4 and extracellular matrix components such as collagen, fibronectin and laminin.

A particularly preferred growth factor will be VEGF. A particularly preferred cell adhesion molecule will be vascular endothelial cell adhesion molecule-1 (VCAM-1).

The person skilled in the art will also be aware that fragments of the aforementioned molecules can be used as probe molecules as long as such fragments provide the necessary binding specificity.

The association of the at least one carrier structure and at least one probe molecule may be achieved by covalent or non-covalent interaction between the carrier structure and the probe molecule. In case of a non-covalent interaction, it is nevertheless intended that the carrier structures in accordance with the present invention do not significantly release the probe molecules during the time periods over which the methods in accordance with the invention are typically performed. Thus, it is currently not envisaged that the carrier structures should release the probe molecules to e.g. establish a signal gradient of the probe molecule. Rather, a substantially permanent association of the probe molecule and the carrier structure is intended.

In one of the preferred embodiments, the probe molecule may be covalently attached to the carrier structure. This may be achieved by functionalizing e.g. the surface of the carrier structures, which preferably are microcarrier structures, or microcarrier beads as has been described above for ECM scaffold forming proteins. The same applies with respect to the coupling chemistry. One may thus e.g. coat a microcarrier bead with a self-assembling polymer. Such a self-assembling polymer may e.g. be polyethylenglycol. The polymers may be further functionalised to introduce chemical functionalities that allow e.g. covalent coupling of an antibody to the surface of the microcarrier bead.

If e.g. growth factors or cell adhesion molecules such as VEGF and VCAM-1 are used as a probe and should be covalently coupled to the microcarrier beads, beads may be used that provide functional groups such as carboxyl groups, amine groups, hydroxyl groups, sulfhydryl groups etc. These groups may then be cross-linked either directly to the growth factors or cell adhesion molecules such as VEGF and VCAM-1 or using a linker that may be homo-or hetero-bifunctional.

Thus, microcarrier beads may be activated for providing a chemical linkage to the probe molecules by e.g. coating the beads with a polymer having e.g. acyl fluoride functionalities. Other covalent attachment chemistries include but are not limited to anhydrides, epoxides, aldehydes, hydrazides, acyl azides, aryl azides, diazo compounds, benzophenones, carbodiimides, imidoesters, isothiocyanates, NHS esters, CNBr, maleimides, tosylates, tresyl chloride, maleic acid anhydrides and carbonyldiimidazole.

As has been mentioned above, a particular preferred type of probe molecule that can be used to modify a microcarrier bead are molecules that activate adhesion of a specific cell type. Thus, in a particularly preferred embodiment, the present invention makes use of microcarrier beads, which are modified to display VEGF and/or VCAM-1 besides the ECM scaffold forming proteins. The function of VEGF has been mentioned above. The cell adhesion molecule VCAM-1 is known to specifically bind leukocytes, thus allowing for separation of white blood cells from plasma, platelets and red blood cells in whole blood samples. VCAM-1 expression is also increased in coronary artery diseases.

The person skilled in the art will be able to identify further suitable probe molecules that are known to detect e.g. specifically a certain cell type. A person skilled in the art will furthermore be capable of identifying suitable attachment chemistry for either covalently or non-covalently associating such a probe molecule with microcarrier beads.

For functionalising of microcarrier beads with e.g. VEGF or VCAM-1, one can rely on typical polystyrene beads of approximately 150 µm diameter. VEGF or VCAM-1 can then be coupled to the surfaces of such beads, which have been functionalised with a reactive group such as e.g. succinimide esters. The succinimide esters will preferably react with primary amines within VEGF or VCAM-1 to form a covalent linkage.

If such microcarrier beads are suspended in a mixture with whole blood or other monocytes and lymphocytes-containing mixtures, this will result in the specific binding of e.g. monocytes and monocytes to the surface of the functionalised microcarrier surfaces depending on the nature of the probe molecule.

As mentioned, the association of the at least one carrier structure with the ECM scaffold forming proteins and the at least one probe molecule may be achieved by covalent or non-covalent interaction between the carrier structure, the ECM scaffold forming proteins and the probe molecule. In case of a non-covalent interaction, it is nevertheless intended that the carrier structures in accordance with the present invention do not significantly release the ECM scaffold forming proteins and the probe molecules during the time periods over which the methods in accordance with the invention are typically performed. Thus, it is currently not envisaged that the carrier structures should release the probe molecules to e.g. establish a signal gradient of the probe molecule or to release the ECM scaffold forming protein. Rather, a substantially permanent association of the ECM scaffold forming protein and probe molecule with the carrier structure is intended.

As pointed out above, at least one carrier structure, which is associated with at least one probe molecule and an ECM, scaffold may be contacted with at least one cell.

The cells that are to be analyzed in accordance with the methods of the present invention are cell types that are known to be involved in the development of certain diseases such as coronary artery diseases, cancer, inflammatory diseases, diabetes etc. Typically, the cells will be recruited by a specific cellular factor during the development of the disease to the site of the disease and exert their function. The cell will further be capable of displaying an ECM scaffold degrading activity by e.g. secreting matrix metalloproteinases or ECM proteases.

Cells in accordance with the invention may be selected from the group comprising embryonic, fetal or adult stem cells, progenitor cells, blood cells, fibroblasts, tumor cells and neuronal cells. For the purposes of the present invention, the use of blood cells is preferred. Typically these blood cells comprise B and T lymphocytes, monocytes, macrophages such as tumor associated macrophages, neutrophils, basophils and eosinophils.

Such cells may further include neutrophils, endothelial cells, smooth muscle cells etc.

As has been mentioned above, the methods of the present invention may be used in the context of diagnosing a disease. As pointed out above for the cells to be determined by the methods in accordance with the invention, the diseases to be diagnosed should be diseases for which an implication of a specific cell type is known and which are characterized in that the specific cell type is recruited during disease development by specific factors that may be used in the context of the present invention as the probe molecule.

Typically, diseases in the context of the present invention include e.g. coronary artery diseases such as atherosclerosis and arteriolosclerosis, diabetes, inflammatory diseases such as rheumatoid arthritis, and different types of cancers such as colorectal, lung, prostate and breast cancer. Other cancers may include leukemia, lymphoma, head and neck cancer, non-small cell lung cancer, ovarian cancer, urinary bladder cancer, kidney cancer, cervical cancer etc.

Other diseases, which are thought to involve cells secreting matrix metalloproteinases, include rheumatoid arthritis and osteoarthritis. Furthermore, cells secreting matrix metalloproteinases are thought to play a role in the processes of morphogenesis, angiogenesis, tissue repair, xerosis and metastasis, autoimmune disorders, allergies, blood and clotting diseases. Diseases characterized by those activities may thus be also illegible for the methods of diagnosis and data acquisition in accordance with the present invention.

Particularly for coronary artery diseases such as atherosclerosis and certain types of cancer, it is known that blood cells such as monocytes and macrophages play a major contributory role during disease development. For example in atherosclerosis, monocytes are recruited by cellular factors such as VEGF and VCAM-1 and then invade into the epithelium. By complex mechanism the monocytes ultimately turn into foam cells, which may contribute to plaque rupture. Similarly, in certain types of cancer, tumour formation is characterised by an aberrant increase of angiogenic activity that is necessary in order to ensure that the tumour receives sufficient blood supply. Furthermore, in order to metastisize, a tumor must "break free" from its environment. This is accomplished by ECM degradation. Both angiogenic and ECM degradation properties can be allocated to monocytes/macrophages, neutrophils and endothelial progenitor cells (EPCs).

In a preferred embodiment, the above described methods in accordance with the invention are thus used for the diagnosis of coronary artery diseases such as atherosclerosis and cancers such as solid tumor types by determining the difference of the extent by which blood cells derived from a patient and a healthy individual act on the ECM scaffold.

A particularly preferred embodiment of the present invention relates to application of the aforementioned methods for diagnosing diseases such as coronary artery diseases including atherosclerosis. A particularly preferred embodiment of the present invention relates to the use of carrier structures that comprise an ECM scaffold and which are further functionalized with molecular probes being growth factors or cell adhesion molecules including VEGF and VCAM-1.

The advantages of the present invention are that such carrier structures can be used to purposively attract and immobilize blood cells such as monocytes, macrophages or the other aforementioned cells to the carrier structures and to then subsequently determine the influence of the ECM degrading enzymes being present and/or secreted by those cells on the ECM scaffold. As already mentioned above, the ECM scaffold and the at least one probe molecule may be associated with the carrier structures by different approaches. Thus, both the scaffold and the probe molecule may be covalently attached to the carrier structures. In other embodiments either the ECM scaffold or the probe molecule may be covalently attached to the carrier structures. In yet other embodiments of the present invention both the scaffold and the probe molecule may be non-covalently associated with the carrier structures.

In the following, the method of diagnosis and method of data acquisition will be discussed with respect to the various steps for the specific case that a microcarrier bead is used which comprises an ECM scaffold being made from e.g. collagen or any of the other aforementioned ECM scaffold forming proteins and which have been additionally functionalized with growth factors or cell adhesion molecules such as VEGF and VCAM-1. The person skilled in the art is, however, clearly aware that the aspects that will be discussed in the following with respect to the specific embodiment may also equally applied for other cell types, other probe molecules and other carrier structures than microcarrier beads.

Once one has provided the microcarrier beads with an ECM scaffold which may be made e.g. of collagen and which have functionalized with e.g. a cell adhesion molecule such as VCAM, these beads are contacted with sample comprising the cells to be analyzed. Such samples may be pre-purified. Pre-purification can help to improve the quality of the methods in accordance with the invention. A typical sample will be derived from a blood sample of a human or animal being. One may e.g. pre-purify those samples by performing a buffy coat centrifugation in order to obtain a fraction of mononuclear cells comprising inter alia monocytes. The samples may then be mixed with the microcarrier beads being functionalized in the above-mentioned matter. One may use also non-fractionated whole blood.Incubation of the above bead structures and the sample may be undertaken over a time period that is typically required to ensure an efficient adherence of the cells in question to the carrier structures. Typical time range in the case of growth factors or cell adhesion molecules such as VEGF and VCAM-1 will be in the range of 5 to 120 minutes. However, incubation times of approximately 10 to 60 minutes and about 20 to 30 minutes may also be appropriate. Following incubation, the cells in question being e.g. blood cells such as monocytes or macrophages will adhere to the microcarrier structures. Then the cell adhered microcarrier structures may be separated from unbound cells by washing the carrier structures, centrifuging the carrier structures or applying other common separation techniques.

One can then determine the extent of the influence of the adhered cells on the stability of the ECM scaffold.

For detecting the extent of influence of the adhered cells on the ECM scaffold various detection approaches are available to the person skilled in the art. Generally, one may use a detectable label or marker that is either covalently or non-covalently attached to the components of the ECM scaffold.

Adhered cells such as monocytes will release the ECM proteases that will degrade the ECM scaffold over time leading to a degradation and release of ECM scaffold forming components. This will also lead to a release of components comprising a detectable label and the extent of the influence of the adhered cells on the stability of the ECM scaffold may thus be measured by determining the release of the components of the ECM scaffold that carry a detectable marker. There are various detectable markers available to the person skilled in the art.

Detectable markers may include fluorescence markers but also other markers that become detectable upon a chemical reaction. Thus, markers may rely on enzymatic reactions by which a staining is produced. For example, horseradish peroxidase may be coupled to components of the ECM scaffold and later on release of the scaffold forming components may be monitored by initiating a reaction through providing the corresponding substrates leading to a staining. Such enzymatic markers may further comprise alkaline phosphatase or chemiluminescent systems.

Further examples of detectable markers, which may also be designated as reporter molecules include but are not limited to dyes, chemiluminescent compounds, metal complexes, magnetic particles, biotin, haptens, radiofrequency transmitters and radioluminescence compounds.

However, the use of fluorescent markers may be preferred as the release of fluorescent markers bearing ECM scaffold components may be easily monitored spectroscopically. Typical fluorescent markers can include Cy5, Cy3, Texas Red, FITC, Attodye, Cydye, Alexa647, Alexa 488, Alexa 546, Alexa 546, Alexa 594, Alexa 633, GFP, YFP, CFP and dsRED. A particularly suitable fluorophore is the life-cell-tracker from Invitrogen. Other markers may be Q-dots or nanoparticles.

The markers may be covalently attached to the components of the ECM scaffold forming components using the coupling chemistries that have been described above in the context of covalently attaching either the probe molecules to the carrier structures or these ECM scaffold forming components to the carrier structures.

For these purposes homo and/or hetero-bi and/or multifunctional cross linking agents may also be used. Typical cross-linking agents include but are not limited to bis (sulfosuccinimid) bis (diazo-benzidine), Dimethyl Adipimidate, Dimethyl Pimelimidate, Dimethyl Suberaimidate, Disuccininnclyl Suberate, Glutaraldehyde, in-Maleimidobenzoyl-N-Hydroxysuccinimide, Sulfosuccinidyl 4-(N-Maleimidomethyl) Cyclohexane-1-carboxylate etc.

If the ECM scaffold comprises components that have been linked to a fluorescent marker, degradation of the ECM scaffold may thus be followed spectroscopically. Once the cells to be analyzed have associated with the carrier structures and the ECM proteases have started to degrade the ECM scaffold, the fluorescently labeled components will be released which will result in different absorption and emission characteristics compared to the situation where they form part of the ECM scaffold.

Detection of the influence of the cells on the stability of the ECM matrix in a preferred embodiment may be determined using Förster Resonance Energy Transfer (FRET). FRET is based on the use of different fluorophores wherein one fluorophore acts as the donor fluorophore while the other fluorophore acts as an acceptor fluorophore. For detection typically the double-labeled species are excitated with a wavelength that is absorbed by the donor fluorophore. The donor fluorophore then transfers the energy to the acceptor fluorophore, which emits light at a different wavelength from the incident light of the donor fluorophore, if the donor and acceptor fluorophores are sufficiently close proximity.

Thus, in one embodiment of the present invention a donor fluorescent label may be attached to the components of the ECM while an acceptor fluorescent label may be attached to the cells, the probe molecules or the carrier structure. In one embodiment, both the donor and acceptor fluorophores can be linked to the ECM scaffold. Before cells from the sample have bound to the carrier structure and started to secrete ECM proteases one will illuminate the system with light of a wavelength at donor absorption wavelength and will observe a strong emission at the acceptor wavelength. Upon degradation of the ECM scaffold, a decrease in the emission at the acceptor wavelength will be observed given that upon release of the labeled component the energy transfer from the donor fluorescent marker to the acceptor fluorescent marker cannot occur any longer due to the spatial separation.

Thus, the present invention in a preferred embodiment relies on FRET technology to determine the extent of the influence of the bound cell on the structure of the ECM scaffold.

A person skilled in the art is aware that for a FRET application the donor and acceptor fluorophores do not necessarily have to be linked as mentioned above. The main requirement is that both fluorophores are in sufficient close proximity to allow for the energy transfer. Thus, a FRET-based detection approach may also be used if the donor and acceptor fluorophores are linked e.g. to components of the ECM scaffold and the probe molecule, or to the ECM scaffold and the carrier structure. One can also fluorescent label the bead prior to the ECM coating. This can also be done with the chemistries listed above. Other embodiments will be apparent to the person skilled in the art.For labeling one may use e.g. the fluorescent markers mentioned above. Appropriate FRET pairs can be Alexa 488 and 546, or Alexa 546 and 594.

The present invention not only relates to methods of diagnosis and data acquisition as mentioned above but also to methods that allow to identify cells as to their capacity of degrading an ECM scaffold, i.e. to provide for matrix metalloproteinase activity. In one embodiment the invention thus pertains to a method wherein the influence of said at least one cell on said ECM scaffold is determined by measuring the degradation of the ECM scaffold by ECM proteases of said at least one cell. In this aspect of the present invention the carrier structures do not necessarily have to provide for a probe molecule that is capable of specifically interacting with the cell within a sample. Thus, the invention relates also to a method of identifying ECM scaffold degrading activities comprising at least the following steps:
a) Providing at least one carrier structure wherein said carrier structure comprises an extra cellular matrix (ECM) scaffold;
b) Providing at least one sample comprising at least one cell that is capable of adhering to said carrier structure;
c) Contacting said at least one sample with said at least one carrier structure;
d) Determining the extent of the influence of said at least one cell being associated with said at least one carrier structure on the stability of the ECM scaffold.

However, carrier structures, which beside an ECM scaffold also comprise such probe molecules may be preferred as in this case it would be possible to purposively select a certain cell type and then analyze whether the cell type has ECM protease activity. The above-described preferred embodiments of the invention as to the type of cells to be analyzed, to the type of probe molecules to be used and to the type of detection methods for analyzing the influence of the cells on the ECM scaffold stability may apply also for these embodiments of the present invention. However, other cell types such as microorganisms may be analyzed.

One advantage of the present invention is that the above-described methods of data acquisition and diagnosis are a valuable approach for analyzing whether a human or animal subject is suffering from disease development or is prone to likely suffer from such a disease. Without being wanted to be bound to a scientific theory it is assumed that the capacity of cells that are involved in the development of a disease to degrade the ECM scaffold differ in the disease versus healthy state. Thus, in the case of atherosclerosis, monocytes derived from a patient suffering from atherosclerosis are likely to provide an ECM protease activity profile that differs from the ECM protease activity observed for monocytes derived from a healthy human or animal being. Accordingly, the influence on the stability of the ECM scaffold in the patient versus the healthy individual can be used to diagnose diseases such as the aforementioned diseases with coronary artery diseases such as atherosclerosis and solid tumor metastasis in cancer being preferred.

One advantage of the present invention is that the ECM scaffold being disposed on the carrier structures can be purposely manipulated to mimic the composition of an ECM scaffold as observed for a specific disease type as closely as possible. Thus, by measuring the influence of a specific cell type on the stability of an ECM scaffold independence on the type of components forming the ECM scaffold it may be possible to not only discriminate for a specific disease between ill versus healthy, but also to make an evaluation of the disease state.

Furthermore, without being wanted to be bound to a scientific theory it is assumed that the association behavior of such cells as the above-described one to the probe molecules being disposed on the carrier structures also allow one to make a decision whether a person is suffering from a specific disease or not. One may then also be in a position to stage the risk associated with the disease. Disease staging and prognosis may rely on such an assay, along with other tests, includinge.g. cholesterol tests for coronary artery diseases with family history. Thus, for coronary artery diseases such as atherosclerosis but also for certain type of cancers, the association behavior of blood cells such as monocytes to carrier structures may differ depending on whether such cells have been derived from a patient or a healthy individual. By determining the association behavior of such cells to carrier structures that have been modified with different amounts of the same probe molecule one can obtain a profile of the amount of cells per carrier structure versus the amount of probe molecule being bound to the carrier structure. Thus, as far as e.g. coronary artery diseases or certain types of cancers are concerned it is possible to determine the amount of blood cells such as monocytes being bound to carrier structures such as microcarrier beads versus the amount of growth factors or cell adhesion molecules such as VEGF and VCAM-1 being bound to carrier structures such as microcarrier beads. If these profiles obtained by this approach are then combined with the findings that the influence of said cells on the stability of the ECM scaffold it may be possible to arrive at an even more accurate diagnosis. One may also use the methods decribed herein for kinetic studies.

While the above invention has been described with respect to some of its preferred embodiments, this is in no way to limit the scope of the invention. The person skilled in the art is clearly aware of further embodiments and alterations to the previously described embodiments that are still within the scope of the present invention.

The following hypothetical examples describe the basic concepts of the present invention.

### EXAMPLES

### Example 1:

Microcarrier bead available from Solohill having a size of approximately 50 to 200 µm size and being coated with collagen are functionalized with VCAM-1. To this end an amount of typically 100 ug/ml to 1 mg/ml may be used. Coupling may be achieved using standard protein cross linking. Subsequently the components of the ECM scaffold are labeled with a fluorescent marker such as Alexa 488.

In a next step these microcarrier beads are then mixed with a heterogeneous cell mixture obtained from human or animal blood. Then the beads are incubated with the sample for about 10 to 20 minutes to allow monocyte adhesion. Subsequently unbound cells are washed away. Then the monocyte adhered microcarrier beads are incubated for e.g. 10 minutes to 2 hours and the change of fluorescence is measured for various time points. In this way, degradation of the ECM scaffold can be followed. This principle procedure is depicted in Fig. 1.

### Experiment 2:

Microcarrier beads are manufactured and functionalized as described in example 1, however in this case a donor and acceptor fluorophore are coupled to the ECM scaffold.

Then the microcarrier beads are again incubated with the blood sample for the above-mentioned time range, unbound cells are removed and degradation of the ECM scaffold is measured over a period of approximately 10 minutes to 2 hours. Given that a FRET approach is taken in this aspect of the invention one should observe a decreasing acceptor emission over time with ECM matrix degradation proceeding.

## Claims

1. Method of diagnosing and/or staging a disease comprising at least the following steps:
a) Providing at least one carrier structure wherein said carrier structure comprises an extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold;
e) Deciding on the occurrence and/or stage of the disease in question based on the data obtained in step d).

2. Method of data acquisition comprising at least the following steps:
a) Providing at least one carrier structure wherein said carrier structure comprises a extra cellular matrix (ECM) scaffold and is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of the influence of said at least one cell being associated with said at least one probe molecule on the stability of the ECM scaffold.

3. Method according to claim 1,
wherein said disease is selected from the group comprising coronary artery diseases, cancer, diabetes and inflammatory and autoimmune diseases or to monitor angiogenesis for regenerative medicine purposes.

4. Method according to any of claims 1 to 3,
wherein said at ECM scaffold covers at least a part of said at least one carrier structure and wherein said at least one probe molecule is associated with at least a part of said ECM scaffold.

5. Method according to any of claims 1 to 3,
wherein said at least one carrier structure is a microcarrier bead.

6. Method according to any of claims 1 to 5
wherein said at least one probe molecule is selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycans.

7. Method according to claim 6,
wherein said ECM matrix is formed from collagen, fibronectin, fibrins, laminins, elastins and/or nidogens.

8. Method according to any of claims 1 to 7,
wherein said at least one cell is selected from the group comprising embryonic, fetal or adult stem cells, progenitor cells, blood cells, fibroblasts, tumor cells and neuronal cells.

9. Method according to claim 8,
wherein said at least one cell is selected from the group of blood cells.

10. Method according to any of claims 1 to 9,
wherein said at least one probe molecule is VCAM, wherein said at least one cell is selected from monocytes and wherein the disease to be diagnosed is atherosclerosis.

11. Method according to any of claims 1 to 10,
wherein the influence of said at least one cell on said ECM scaffold is determined by measuring the degradation of the ECM scaffold.

12. Method according to any of claims 1 to 11,
wherein the influence of said at least one cell on said ECM scaffold is determined by FRET.
